Europäisches Patentamt

European Patent Office

Office européen des brevets

(19)

(11) Veröffentlichungsnummer: **0 151 724**
**B1**

(12) # EUROPÄISCHE PATENTSCHRIFT

(45) Veröffentlichungstag der Patentschrift:
**19.11.87**

(51) Int. Cl.⁴: **A 61 F 2/38**

(21) Anmeldenummer: **84114800.0**

(22) Anmeldetag: **05.12.84**

(54) **Tibiateil für eine Kniegelenkprothese.**

(30) Priorität: **10.02.84 CH 648/84**

(43) Veröffentlichungstag der Anmeldung:
**21.08.85 Patentblatt 85/34**

(45) Bekanntmachung des Hinweises auf die Patenterteilung:
**19.11.87 Patentblatt 87/47**

(84) Benannte Vertragsstaaten:
**AT DE FR GB IT**

(56) Entgegenhaltungen:
**EP - A - 0 005 612**
**DE - A - 3 314 038**
**GB - A - 2 005 545**
**GB - A - 2 120 943**

(73) Patentinhaber: **GEBRÜDER SULZER
AKTIENGESELLSCHAFT, Zürcherstrasse 9,
CH-8401 Winterthur (CH)**

(72) Erfinder: **Horber, Willi, Turbinenstrasse 12,
CH-8005 Zürich (CH)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

## Beschreibung

Die Erfindung betrifft einen Tibiateil für eine Kniegelenkprothese, in deren tibialem Plateau Verankerungszapfen für eine Verankerung im Knochen vorgesehen sind, während die dem Plateau gegenüberliegende Oberfläche als tibiale Lagerfläche ausgebildet ist, welche Verankerungszapfen über mindestens eine, zu den Zapfenachsen schrägflächige Erweiterung in das Plateau übergehen.

Die Druckschrift «ICLH-Knieprothesen» der Firma Protek AG, Bern, zeigt Prothesenkonstruktionen, bei denen das der Tibia zugewandte Plateau als ebene, senkrecht zu den Verankerungszapfen stehende Fläche ausgebildet ist, da für eine solche Plateau-Form die Operationstechnik relativ einfach ist. In der Praxis hat sich jedoch gezeigt, dass diese Tibiateile, die nur mit den Verankerungszapfen in das Schienbein eingreifen, mit ihrem Plateau jedoch lediglich eben aufliegen, Belastungen, die eine Komponente parallel zur Plateau-Ebene aufweisen, nur relativ schlecht aufnehmen können; denn diese «Scher»-Komponente kann dabei nur vom relativ kleinen Umfang der Verankerungszapfen übernommen werden, wodurch dort relativ hohe spezifische Belastungen mit Belastungsspitzen entstehen, die zu einer Rückbildung des Knochengewebes führen können.

Tibiateile der eingangs genannten Art sind bekannt aus der DE-A-33 14 038; bei dieser Konstruktion ist der Tibiateil, der teilweise im Tibiaknochen «versenkt» ist, ebenfalls mit zwei Verankerungszapfen versehen. Der untere Sockelbereich, der die Ansatzstellen der Verankerungszapfen mit einem plattenartigen Auflagekörper für eine Kunststoffauflage verbindet, hat dabei die Form eines trogähnlichen prismatischen Körpers, der sich zum Plateau des Auflagekörpers schrägflächig zur Zapfenachse erweitert. Zwar verbessert der trogartige Sockelbereich die Widerstandsfähigkeit der Verankerung gegen seitliche Scherkräfte gegenüber der vorstehend diskutierten Konstruktion; die Belastung des Knochens durch diese Kräfte ist jedoch über den Umfang des Implantates stark unterschiedlich, so dass nach wie vor örtlich hohe Belastungsspitzen auftreten können.

Weiterhin ist die Operationstechnik durch die trog-ähnliche Form des bekannten Tibiateils erheblich erschwert. Aufgabe der Erfindung ist es, einen Tibiateil zu schaffen, bei dem die von der Seite, d.h. nicht in Richtung der Achse wirkenden Kräfte grossflächig, möglichst gleichmässig über den Umfang des Tibiateils verteilt auf die Tibia übertragen und so Belastungsspitzen abgebaut werden.

Gemäss der vorliegenden Erfindung wird diese Aufgabe dadurch gelöst, dass die Erweiterung die Form eines zur entsprechenden Zapfenachse koaxialen Rotationskörpers, beispielsweise eines koaxialen Kegelstumpfes, aufweist.

Die Ausbildung der Erweiterungen als Rotationskörper vergleichmässigt die Verteilung der Seitenkräfte längs ihres Umfanges und erfordert gegenüber einem ebenen Schnitt nur relativ geringfügig vergrösserte Fräsarbeit während der Implantation; die kegelstumpfförmige Ausbildung lässt dabei die Fräsarbeit für den Operateur besonders einfach werden.

Im Sinne einer verbesserten Übertragung der Seitenkräfte auf die proximale Tibia ist es unter Umstän-den zweckmässig, wenn zwischen Verankerungs-zapfen und Plateau mindestens zwei schrägflächige Erweiterungen vorgesehen sind, von denen die proximale einen grösseren Öffnungswinkel gegen die Zapfenachse aufweist als die distale; dabei kann die distale Erweiterung mit der Zapfenachse einen Win-kel von $\alpha < 45°$, insbesondere von 30°, bilden, während der entsprechende Winkel für die proximale Erweiterung $\beta < 90°$, insbesondere bei 75°, liegen kann. Schliesslich ist es auch möglich, dass die schrägflächige Erweiterung in einer stetigen Kurve aus der Vertikalen in die Horizontale übergeht.

Wegen der günstigen Gleiteigenschaften besteht der neue Tibiateil vorzugsweise aus Kunststoff, z.B. Polyäthylen; ein aus Kunststoff gefertigter Tibiateil hat darüberhinaus den Vorteil, dass der Plattenkörper durch Zuschneiden an die Grösse der «abzudeckenden Tibiafläche» abgepasst werden kann, denn es ist unter Umständen erwünscht, dass der Tibiateil die Tibiaoberfläche ohne überstehenden Rand genau abdeckt. Es ist jedoch auch möglich, den neuen Tibiateil aus einem beliebigen anderen der bekannten Implantatwerkstoffe — wie Metalle, Metall-Legierung, biokeramische Werkstoffe, Kohlenstoff oder faserverstärkte Verbundwerkstoffe — herzustellen oder ihn aus zwei oder mehr dieser Werkstoffe zusammenzusetzen. Weiterhin können seine Oberflächen ganz oder teilweise mit bioinerten oder bioaktiven Beschichtungen versehen sein.

Im folgenden wird die Erfindung anhand von Ausführungsbeispielen im Zusammenhang mit der Zeichnung erläutert.

Fig. 1 ist eine Anterior/posterior-Ansicht des neuen Tibiateils;

Fig. 2 gibt eine Ansicht von Fig. 1 von unten wieder, während

Fig. 3 eine Ansicht von Fig. 1 von links ist;

Fig. 4 ist in gleicher Darstellung wie Fig. 1 der rein schematische Aufbau einer weiteren Ausführungsform des neuen Tibiateils.

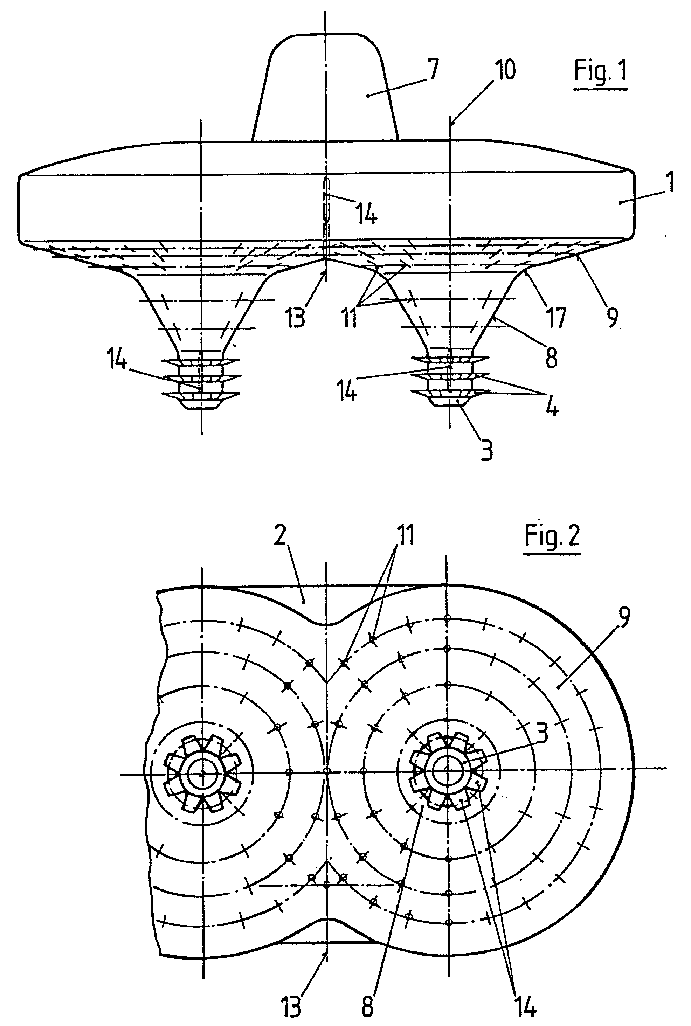

Der in Fig. 1 gezeigte Tibiateil besteht im wesentlichen aus einem Plattenkörper 1, der an seine, dem Knochen zugewandten Tibia-Plateau 2 (Fig. 2) Verankerungszapfen 3 aufweist; diese sind mit verformbaren, zirkularen Rippen 4 versehen und werden bei der Implantation in entsprechenden Bohrungen des Tibiaknochens verankert.

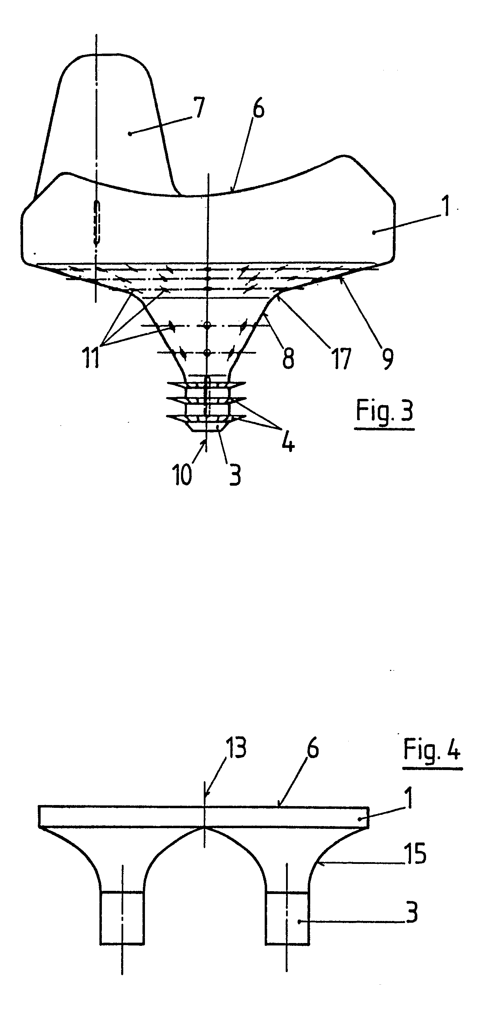

Die Oberseite des Plattenkörpers 1 weist tibiale Lagerflächen 6 (Fig. 3) auf, auf denen die kondylen-ähnlich geformten Lagerflächen des nicht gezeigten Femurteils gleitend abrollen können. Zwischen den Lagerflächen 6 erhebt sich, nach posterior versetzt, ein Führungszapfen 7, durch den der Femurteil bei seiner gleitenden Abrollbewegung geführt ist.

Erfindungsgemäss ist der Übergang von den Verankerungszapfen 3 zum Tibia-Plateau 2 als schrägflächige Erweiterungen 8 und 9 ausgebildet, die — im Ausführungsbeispiel nach Fig. 1-3 — als mit den zugehörigen Verankerungszapfen koaxiale Kegelstümpfe gestaltet sind. Weiterhin sind bei der genannten Ausführung jedem Verankerungszapfen 3 eine distale Erweiterung 8 und eine proximale Erweiterung 9 mit abgerundetem Übergang 17 zugeordnet, wobei die distale Erweiterung 8 mit der Zapfenachse 10 einen Winkel $\alpha$ von 30° bildet, während
der entsprechende Winkel für die proximale Erweiterung 9 $\beta$ 75° beträgt.

Die Schrägflächen der Erweiterungen 8 und 9 sind
mit regelmässig oder unregelmässig verteilten Bohrungen 11 bedeckt, in die im Laufe der Zeit spongioses Gewebe einwächst.

In jeder Zapfenachse 10 sowie in der anterior/
posterior verlaufenden Mittelebene 13 des Plattenkörpers 1 sind kleine Metallstäbchen 14 eingebettet,
die eine Bestimmung der Lage dieser geometrischen
Orte auf einem Röntgenbild erleichtern.

Die Erweiterungen 8 und 9 sind bei der zweiten
Ausführungsform nach Fig. 4 ersetzt durch stetig
gekrümmte Flächen 15, die ebenfalls koaxial mit je
einem Verankerungszapfen 3 einen Rotationskörper
bilden, an dessen Ende die Zapfen 3 angesetzt sind.


**Patentansprüche**

1. Tibiateil für eine Kniegelenkprothese, in deren
tibialem Plateau (2) Verankerungszapfen (3) für eine
Verankerung im Knochen vorgesehen sind, während
die dem Plateau (2) gegenüberliegende Oberfläche
als tibiale Lagerfläche (6) ausgebildet ist, welche
Verankerungszapfen (3) über mindestens eine, zu
den Zapfenachsen (10) schrägflächige Erweiterung
(8, 9; 15) in das Plateau (2) übergehen, dadurch gekennzeichnet, dass die Erweiterung (8, 9; 15) die
Form eines zur entsprechenden Zapfenachse (10)
koaxialen Rotationskörpers, beispielsweise eines
koaxialen Kegelstumpfes, aufweist.

2. Tibiateil nach Anspruch 1, dadurch gekennzeichnet, dass zwischen Verankerungszapfen (3)
und Plateau (2) mindestens zwei schrägflächige
Erweiterungen (8, 9) vorgesehen sind, von denen
die proximale (9) einen grösseren Öffnungswinkel
gegen die Zapfenachse (10) aufweist als die distale
(8).

3. Tibiateil nach Anspruch 2, dadurch gekennzeichnet, dass die distale Erweiterung (8) mit der
Zapfenachse (10) einen Winkel $\alpha < 45°$, insbesondere von 30°, bildet.

4. Tibiateil nach Anspruch 3, dadurch gekennzeichnet, dass die proximale Erweiterung (9) mit der
Zapfenachse (10) einen Winkel $\beta < 90°$, insbesondere von 75°, bildet.

5. Tibiateil nach Anspruch 1, dadurch gekennzeichnet, dass die schrägflächige Erweiterung (15) in
einer stetigen Kurve aus der Vertikalen in die Horizontale übergeht.


**Claims**

1. A tibia part for a knee joint replacement having in its tibial plateau (2) anchor pins (3) for an
anchorage in the bone, whereas the surface opposite
the plateau (2) is a tibial bearing surface (6), the
anchor pins (3) merging into the plateau (2) by way
of at least one widening (8, 9; 15) whose surface is
at an inclination to the pin axes (10), characterised
in that the widening (8, 9; 15) has the shape of a body
of rotation, for example, a coaxial frustum, coaxial
of the corresponding pin axis (10).

2. A tibia part according to claim 1, characterised
in that at least two inclined-surface widenings (8, 9)
are present between the anchor pins (3) and the
plateau (2), the proximal widening (9) having a
greater opening angle to the pin axis (10) than the
distal widening (8).

3. A tibia part according to claim 2, characterised
in that the distal widening (8) forms an angle $\alpha <
45°$, more particularly 30°, with the pin axis (10).

4. A tibia part according to claim 3, characterised
in that the proximal widening (9) forms an angle $\beta <
90°$, more particularly 75°, with the pin axis (10).

5. A tibia part according to claim 1, characterised
in that the inclined-surface widening (15) merges
from the vertical into the horizontal by way of a
continuous curve.


**Revendications**

1. Partie tibiale pour une prothèse rotulienne,
dans le plateau tibial (2) de laquelle des tenons
d'ancrage (3) sont prévus pour un ancrage dans l'os,
tandis que la surface tournée à l'opposé du plateau
(2) est réalisée sous la forme d'une surface (6) de
support tibial, lesdits tenons d'ancrage (3) fusionnant dans le plateau (2) par l'intermédiaire d'au
moins un évasement (8, 9; 15) à surface inclinée par
rapport aux axes (10) des tenons, caractérisée par le
fait que l'évasement (8, 9; 15) présente la forme d'un
corps en rotation coaxial à l'axe correspondant (10)
du tenon, par exemple d'un tronc de cône coaxial.

2. Partie tibiale selon la revendication 1, caractérisée par le fait que, entre les tenons d'ancrage (3) et
le plateau (2), sont prévus au moins deux évasements (8, 9) à surfaces inclinées, parmi lesquels
l'évasement proximal (9) présente, par rapport à
l'axe (10) du tenon, un angle d'ouverture plus grand
que celui de l'évasement distal (8).

3. Partie tibiale selon la revendication 2, caractérisée par le fait que l'évasement distal (8) forme, avec
l'axe (10) du tenon, un angle $\alpha < 45°$, mesurant
notamment 30°.

4. Partie tibiale selon la revendication 3, caractérisée par le fait que l'évasement proximal (9) forme,
avec l'axe (10) du tenon, un angle $\beta < 90°$, mesurant notamment 75°.

5. Partie tibiale selon la revendication 1, caractérisée par le fait que l'évasement (15) à surface inclinée passe de la verticale à l'horizontale en suivant
une courbure constante.

Fig. 1

Fig. 2

Fig. 3

Fig. 4